(19) Europäisches Patentamt

European Patent Office

Office européen des brevets

(11) EP 0 485 078 B1

(12) EUROPEAN PATENT SPECIFICATION

(45) Date of publication and mention
of the grant of the patent:
**20.11.1996 Bulletin 1996/47**

(51) Int. Cl.$^6$: **B01D 3/14**, B01D 3/42,
C07C 43/04, C07C 41/09,
C07C 41/42

(21) Application number: **91309465.2**

(22) Date of filing: **15.10.1991**

(54) **Process for the production of MTBE**

Prozess zur Herstellung von MTBE

Procédé de production de MTBE

(84) Designated Contracting States:
**BE DE ES FR GB IT NL SE**

(30) Priority: **07.11.1990 US 609755**

(43) Date of publication of application:
**13.05.1992 Bulletin 1992/20**

(73) Proprietor: **CHEMICAL RESEARCH & LICENSING COMPANY**
**Pasadena, Texas 77507 (US)**

(72) Inventors:
• **Smith, Lawrence A., Jr.**
**Houston, Texas 77401 (US)**
• **Arganbright, Robert P.**
**Seabrook, Texas 77586 (US)**

(74) Representative: **Smaggasgale, Gillian Helen et al**
**Mathys & Squire,**
**100 Grays Inn Road**
**London WC1X 8AL (GB)**

(56) References cited:
**EP-A- 0 405 781          GB-A- 2 096 603**
**US-A- 4 918 244**

**Description**

BACKGROUND OF THE INVENTION

Field of the Invention

The present invention relates to the production of methyl tertiary butyl ether from the reaction of methanol and tertiary butyl alcohol. More particularly the invention relates to the concurrent reaction of the methanol and tertiary butyl alcohol and separation of the reaction products from the reactants by fractional distillation wherein the catalyst also acts as a distillation structure.

Related Art

Methyl tertiary butyl ether (MTBE) is a useful component for improving the octane of gasolines and has commonly been prepared by the acid catalyzed reaction of methanol with isobutene. Examples of such a process are disclosed in U.S. patents 4,039,590 and 4,198,530.

Recently a new method of carrying out catalytic reactions has been developed, wherein the components of the reaction system are concurrently separable by distillation, using the catalyst structures as the distillation structures. This method is now generally known as catalytic distillation and any reference to catalytic distillation herein will be taken to mean this method or process. Such systems are described variously in U.S. Patents 4,215,011; 4,232,177; 4,242,530; 4,302,356; 4,307,254; 4,336,407; 4,439,350; 4,443,559; and 4,482,775 commonly assigned herewith.

Briefly, a preferred and commercial catalyst structure described in the above patents comprises a cloth belt with a plurality of pockets spaced along the belt and containing particulate catalyst material, said cloth belt being wound in a helix about a spacing material such as stainless steel knitted mesh. These units are then disposed in the distillation column reactor. In addition, commonly assigned U.S. Patent Nos. 4,443,559 and 4,250,052 disclose a variety of catalyst structures for this use and are incorporated herein.

The success of catalytic distillation lies in an understanding of the principles associated with distillation. Because the reaction is occurring concurrently with distillation, the initial reaction product is removed from the reaction zone as quickly as it is formed which minimizes further reaction. The heat of the reaction, if any, simply creates more boil up, but no increase in temperature.

The production of MTBE has also been accomplished by the reaction of methanol with tertiary butyl alcohol (TBA) in the presence of an acidic catalyst. See for example U.S. Pat. No. 2,282,469, which discloses the production of MTBE using phosphoric acid catalyst to dehydrate tertiary butyl alcohol and methanol in a straight pass reaction at 65.6 to 176.7°C (150 to 350°F) in low yields.

US Pat. No. 3,267,156 discloses the preparation of dialkyl ethers by dehydration over acid resin catalyst at 121.1 to 204.4°C (250 to 400°F.)

U.S. Pat. No. 4,822,921, discloses the production of MTBE using phosphorus on titania catalyst to dehydrate methanol/tertiary butyl alcohol (2:1) in a straight pass reaction at 48.9°C (120°F)+.

U.S. Pat. No. 4,827,048, discloses the production of MTBE using heteropoly acid catalyst on an inert support to dehydrate methanol and tertiary butyl alcohol in a straight pass reaction at 48.9°C (120°F)+.

U.S. Pat. No. 4,918,244 discloses MTBE production by reacting methanol and tertiary butyl alcohol in a rectification tower in a packed bed at .5 to 250 °C using acid resin catalyst by adding TBA and methanol to a flask and refluxing in or distilling them through the catalyst bed. Selectivity to MTBE was around 72% at conversion of 95% Utilizing modeling techniques, Nelson, et al., then suggest a continuous process with methanol being fed below the catalyst bed and TBA being fed above the bed. Because the reactor is in reality a distillation column, the continuous phase contained therein is the vapor. However the etherification reaction proceeds preferentially in the liquid phase The only liquid in a distillation tower reactor is a result of reflux, either internal or external, in the column. The present invention is a process which obtains the advantages of catalytic distillation and liquid phase reactions.

**SUMMARY OF THE INVENTION**

According to the present invention there is provided a process for the production of methyl tertiary butyl ether from the reaction of methanol and tertiary butyl alcohol comprising the steps of:

(a) feeding a feed mixture containing methanol and tertiary butyl alcohol into a feed zone of a distillation column reactor;
(b) concurrently in said distillation column reactor

(i) contacting said feed mixture with a acid ion exchange resin catalyst as a distillation structure component in a distillation reaction zone, thereby reacting at least a portion of said methanol and said tertiary butyl alcohol to form a reaction mixture containing methyl tertiary butyl ether, water, unreacted methanol and unreacted tertiary butyl alcohol, and

(ii) separating said methyl tertiary butyl ether and said unreacted methanol from said unreacted tertiary butyl alcohol and said water by fractional distillation;

(c) withdrawing said methyl tertiary butyl ether and said unreacted methanol from said distillation column reactor as overheads; and

(d) withdrawing said unreacted tertiary butyl alcohol and said water from said distillation column reactor as bottoms and characterized in that a continuous level is maintained in said distillation reaction zone.

Briefly, the invention is a catalytic distillation process for the production of methyl tertiary butyl ether (MTBE) from the reaction of tertiary butyl alcohol (TBA) and methanol (MeOH) by feeding a mixture of the reactants at a preferred molar ratio of methanol to tertiary butyl alcohol of between 1:1 and 3:1 into the reaction distillation column in a feed zone which is preferably below a bed of acid ion exchange resin catalyst which constitutes a reaction distillation zone characterized in that a continuous liquid level is maintained in the catalyst bed. The continuous liquid level preferably covers the catalyst bed. In the reaction distillation zone the reactants contact the acid ion exchange resin catalyst and catalytically react to form MTBE and water. The production rate of the present process is quite high, i.e., higher than a catalytic distillation carried out without the liquid level. The acid ion exchange resin catalyst is in such a form as to act as both the catalyst for the reaction and distillation structure for the fractional distillation. Suitable catalytic distillation structures are the catalyst containing cloth belts described above and disclosed in the aforementioned U.S. patents 4,215,011; 4,302,356 and 4,443,559 to which reference may be had for greater detail on this point.

The overhead pressure is preferably between 25.33 and 2533 kPa (0.25 and 25 atmospheres). The temperature in the distillation reaction zone is preferably between 4.4 and 204.4°C (40 and 400°F), more preferably between 65.6 and 82.2°C (150 and 180°F).

The liquid level in the reaction distillation zone is achieved by a liquid flow restrictor between the distillation reaction zone and the lower distillation zone. That is, the vapor from below may rise up to (and through) the reaction distillation zone as in a conventional or prior operation but a portion of the liquid is maintained there. If a single distillation column reactor is used, a conventional distillation tray with the downcomer area blocked is located between the reaction distillation zone and the distillation zone. A by pass line for liquid flow is provided about the tray and a valve is provided in the liquid flow conduit to restrict liquid downflow and thereby to build up a liquid level above that tray just below the catalyst bed. Alternatively a perforated plate may be used to support the catalyst and cause a liquid pressure drop in the column thus building up a liquid level in the catalyst bed. If the two column system is used, then a valve or other restriction means is placed in the liquid flow line between the two columns.

While the particular position of the liquid level has been described above to be at the lower end of the distillation reaction zone, it could just as easily be placed anywhere in the catalyst bed depending upon the desired reactions.

The term "liquid level" is used herein to mean an increased density of the material in the reaction distillation zone over that of a pure distillation as distinguished from a continuous liquid phase. The phase system as present in the reaction distillation zone is physically a froth. The froth is the result of the vapor traveling up through the liquid retained in the zone. The liquid level or froth may cover the catalyst bed or a portion thereof.

Another way of viewing this is that in normal distillation there is a vapor with liquid (internal reflux) trickling down through the vapor and contacting the catalyst whereas in the present liquid full system the vapor is traveling up through a liquid phase to create the froth or foam. The "frothing" of the instant invention is a result of the flow restrictor and not the upward velocity of vapor as the term flood is commonly understood.

Hence in essence the benefits of the distillation are still obtained, i.e., separating the various components by the distillation whereas the increased liquid volume in contact with the catalyst improves the synthesis reaction.

## BRIEF DESCRIPTION OF THE DRAWING

FIG. 1    is a flow diagram in schematic form of one embodiment of the present invention.

FIG. 2    is a flow diagram in schematic form of another embodiment of the present invention.

FIG. 3    is a plot of MTBE production versus overhead takeoff comparing the "liquid full" operation with normal operation.

DESCRIPTION OF THE PREFERRED EMBODIMENT

1. Catalyst and Distillation Structure

Suitable acid cation exchange resins include those which contain sulfonic acid groups, and which may be obtained by polymerization or copolymerization of aromatic vinyl compounds followed by sulfonation. Examples of aromatic vinyl compounds suitable for preparing polymers or copolymers are: styrene, vinyl toluene, vinyl naphthalene, vinyl ethylbenzene, methyl styrene, vinyl chlorobenzene and vinyl xylene. A large variety of methods may be used for preparing these polymers; for example, polymerization alone or in admixture with other monovinyl compounds, or by crosslinking with polyvinyl compounds; for example, with divinyl benzene, divinyl toluene, divinyl phenylether and others. The polymers may be prepared in the presence or absence of solvents or dispersing agents, and various polymerization initiators may be used, e.g., inorganic or organic peroxides, persulfates, etc.

The sulfonic acid group may be introduced into these vinyl aromatic polymers by various known methods; for example, by sulfating the polymers with concentrated sulfuric and chlorosulfonic acid, or by copolymerizing aromatic compounds which contain sulfonic acid groups (see e.g., US Pat. No. 2,366,007). Further sulfonic acid groups may be introduced into the polymers which already contain sulfonic acid groups; for example, by treatment with fuming sulfuric acid, i.e., sulfuric acid which contains sulfur trioxide. The treatment with fuming sulfuric acid is preferably carried out at 0 to 150° C and the sulfuric acid should contain sufficient sulfur trioxide so that it still contains 10 to 50% free sulfur trioxide after the reaction. The resulting products preferably contain an average of 1.3 to 1.8 sulfonic acid groups per aromatic nucleus. Particularly, suitable polymers which contain sulfonic acid groups are copolymers of aromatic monovinyl compounds with aromatic polyvinyl compounds, particularly, divinyl compounds, in which the polyvinyl benzene content is preferably 1 to 20% by weight of the copolymer (see, for example, German Patent Specification DE-C-908,240). The ion exchange resin is generally used in a granular size of about 0.25 to 1 mm, although particles from 0.15 mm up to about 2 mm may be employed. The finer catalysts provide high surface area, but also result in high pressure drops through the reactor. The macroreticular form of these catalysts have much larger surface area exposed and limited swelling which all of these resins undergo in a non-aqueous hydrocarbon medium compared to the gelular catalysts.

The container employed to hold the catalyst particles may have any configuration, such as the pockets disclosed in the commonly assigned patents above or the container may be a single cylinder, sphere, doughnut, cube, tube or the like.

Each container containing a solid catalytic material comprises a catalyst component. Each catalyst component is intimately associated with a spacing component which is comprised of at least 70 volume % open space up to about 95 volume % open space. This component may be rigid or resilient or a combination thereof. The combination of catalyst component and spacing component form the catalytic distillation structure. The total volume of open space for the catalytic distillation structure should be at least 10 volume % and preferably at least 20 volume % up to about 65 volume %. Thus, desirably the spacing component or material should comprise about 30 volume % of the catalytic distillation structure,preferably about 30 volume % to 70 volume %. Resilient materials are preferred. One suitable such material is open mesh knitted stainless wire, known generally as demister wire or an expanded aluminum. Other resilient components may be similar open mesh knitted polymeric filaments of nylon, teflon and the like. Other materials such as highly open structures foamed material, e.g., reticulated polyurethane foam (rigid or resilient) may be formed in place or applied around the catalyst component.

In the case of larger catalyst components such as from about 6.35 to 12.7 mm (1/4 inch to 1/2) pellets, spheres, pills and the like, each such larger component may be individually intimately associated with or surrounded by the spacing component as described above.

It is not essential that the spacing component, entirely cover the catalyst component. It is only necessary that the spacing component intimately associated with the catalyst component will act to space the various catalyst components away from one another as described above. Thus, the spacing component provides in effect a matrix of substantially open space in which the catalyst components are randomly but substantially evenly distributed.

A preferred catalytic distillation structure for use herein comprises placing the mole sieve or cation exchange resin particles into a plurality of pockets in a cloth belt, which is supported in the distillation column reactor by open mesh knitted stainless steel wire by twisting the two together in a helical form. This allows the requisite flows and prevents loss of catalyst. The cloth may be any material which is inert in the reaction. Cotton or linen are useful, but fiber glass cloth or "Teflon" cloth are preferred.

In the following examples the catalyst packing consisted of bags in the form of a fiber glass cloth belt approximately six inches wide with narrow pockets approximately 19.05 mm (3/4 inch) wide sewn across the belt. The pockets are spaced about 6.35 mm (1/4 inch) apart.

These pockets are filled with the catalyst particles to form approximately cylindrical containers, and the open ends are then sewn closed to confine the particles. This belt is then twisted into a helical form to fit inside the column. Twisted in with the belt is also a strip of an open mesh knitted stainless steel wire, which serves to separate the mole sieve filled cloth pockets and provide a passage for vapor flow.

The wire mesh provides the support for the catalyst belt and provides some degree of vapor passage through the catalyst particles, which otherwise form a very compact bed which has a high pressure drop. Thus, the down flowing liquid is in intimate contact with the rising vapors and the catalyst in the column.

In commercial-scale operations, it is contemplated, catalyst packing would be made up of alternating layers of catalyst filled cloth belts similar to the ones described above, and a spacing material which could be of any convenient, suitable substance, such as a corrugated wire screen or wire cloth or a knitted wire mesh. The layers would be arranged vertically or horizontally. For simplicity of fabrication and for better distribution of vapor flow passages, a vertical orientation is preferred. The height of a section of this packing could be of any convenient dimension, from a few inches to several feet. For ease of assembly and installation, the packing would be made into sections of the desired shape and size, each section fastened together with circumferential bands of tie wires depending on its size and shape. A complete assembly in a column would consist of several sections, arranged in layers, with possibly the orientation of the catalyst-filled belts turned at right angles in successive layers to improve liquid and vapor flow distribution.

2. Process Description

The reaction system can be described as heterogeneous since the catalyst remains as a distinct entity. The catalyst may be employed in such conventional distillation packing shapes, as Rashig rings, Pall rings, saddles or the like. Similarly, the catalyst may be employed in granular or bead form as described herein above.

Bulk type liquid phase reactions have as one problem the control of the temperature. The distillation avoids the problem entirely. Because the reaction is occurring concurrently with distillation, the initial reaction products, MTBE and water, are removed from the reaction zone nearly as quickly as they are formed. This removal of the MTBE is of particular importance because it minimizes decomposition of the MTBE which is catalyzed by the same catalyst. Also because the MTBE is boiling, the temperature of the reaction is controlled by the boiling point of the mixture in the reactor at the system pressure. The heat of the reaction, which is exothermic, simply consumes more boil up, but no change in temperature. That is, if the heat is added in excess, there is no harm done since the excess will only result in more boil up. Third, the reaction has an increased driving force because the reaction products have been removed and cannot contribute to the reverse reaction (Le Chatelier's Principle).

As a result, a great deal of control over the rate of reaction and distribution of products can be achieved by regulating the system pressure. Also, adjusting the throughput ( $residence\ time = liquid\ hourly\ space\ velocity^{-1}$ ) gives further control of product distribution.

A reflux is preferably included in the system. The reflux ratio could vary over the rate of 0.5 to 25:1. In practice, the higher ratio may be used to compensate for a short catalyst bed such as required for experimental work. In commercial size units the catalyst bed would be provided so that lower reflux and hence higher unit productivity could be obtained, e.g., 0.5 to 2:1.

Because the reaction proceeds preferentially in the liquid, it is preferred to have a denser almost completely liquid phase in the catalyst bed. A liquid flow restrictor is thus placed under the catalyst bed as described hereinabove and in commonly assigned co-pending patent application serial number 07/328,487. filed March 23, 1989.

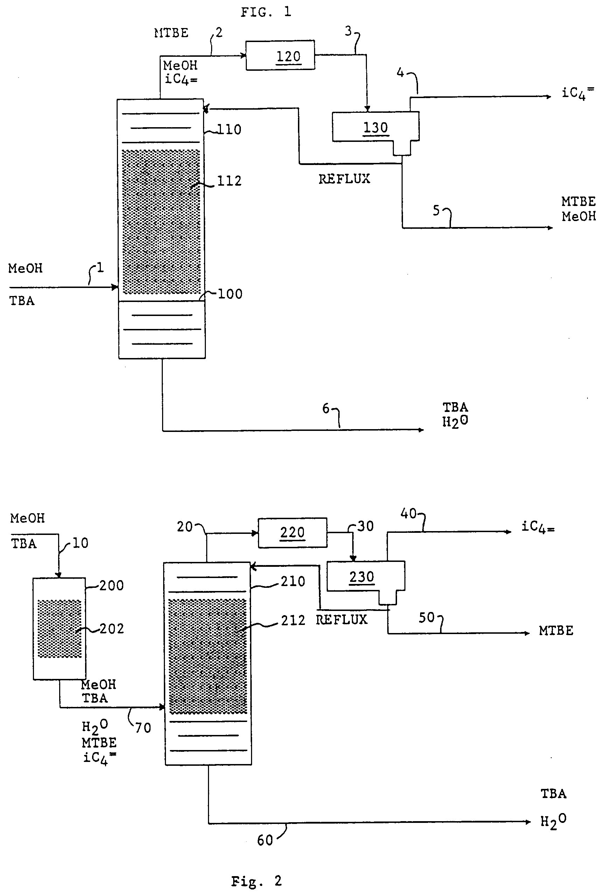

Referring now to the FIG.'s typical flow schemes are shown in simplified schematic form. In FIG. 1 a mixture containing MeOH and TBA are fed to the distillation column reactor 110 below the catalyst bed 112 via flow line 1. The molar ratio of methanol to tertiary butyl alcohol in said feed mixture is preferably between 1:1 and 3:1. The mixture is boiled up into the bed where it contacts the catalyst, preferably an acid ion exchange resin. The flow restrictor 100 builds up a liquid level in the catalyst bed which is frothing due to the rising vapor. The MeOH and TBA preferentially react to form a reaction mixture containing MTBE, water, unreacted MeOH and unreacted TBA. Additionally some isobutene may also be formed. The unreacted MeOH along with the product MTBE and isobutene, if any, is distilled overhead via flow line 2 to partial condenser 120 where the MeOH and MTBE are condensed prior to collection in receiver 130. Any isobutene is separated and removed via flow line 4. The MeOH and MTBE may be removed via flow line 5 for further separation as by water wash. Alternatively a portion of the condensed overhead may be returned to the distillation column reactor as reflux. The unreacted TBA and water product are removed from the bottom of the column via flow line 6 for further separation and recycle of the TBA if desired.

In FIG. 2 a second embodiment is shown in which the initial reaction of the MeOH and TBA is carried out in a conventional downflow fixed bed reactor 200 containing a bed 202 of the same or similar catalyst as is used in the distillation column reactor 210. In this embodiment, the distillation column reactor 210 acts a polishing reactor to react essentially all of the MeOH left in flow line 70 exiting the fixed bed reactor 200.

EXAMPLE 1

The reaction of TBA and MeOH to produce MTBE was carried out in a 25.4 mm (one inch) automated distillation column reactor. The catalyst used was CT-175 cation exchange resin manufactured by Purolite Corp. and was placed into the cloth bags and supported as hereinabove described. 1.83 m (Six feet) of the catalyst were placed into the column with 1.22 m (4 feet) of standard distillation packing above and 1.83 m (6 feet) below the catalyst. The distillation column reactor was operated in both the froth and non froth condition. The efficiency of the process was determined in terms of conversion, production rate and selectivity to MTBE. Production rates of MTBE from TBA and MeOH were in the range considered acceptable for commercial reactions. Complete results along with conditions and flow rates are given in TABLE I following.

In order to provide a quantitative measure of the liquid level employed in the runs in the TABLE the following calibration was established for the one inch column: The column was filled with liquid $C_4$ cut to cover the catalyst bed and the pressure differential (pd) measured across the bed. This was defined as 100%. In the runs reported in the TABLE the pressure differential was reported as a percent of the liquid filled pd. A liquid level according to the present invention in this equipment would have a pd% of at least 50 and generally from about 50 to 70%. The pd% for a catalytic distillation without a liquid level would be less than 10, usually 0 to 10_.

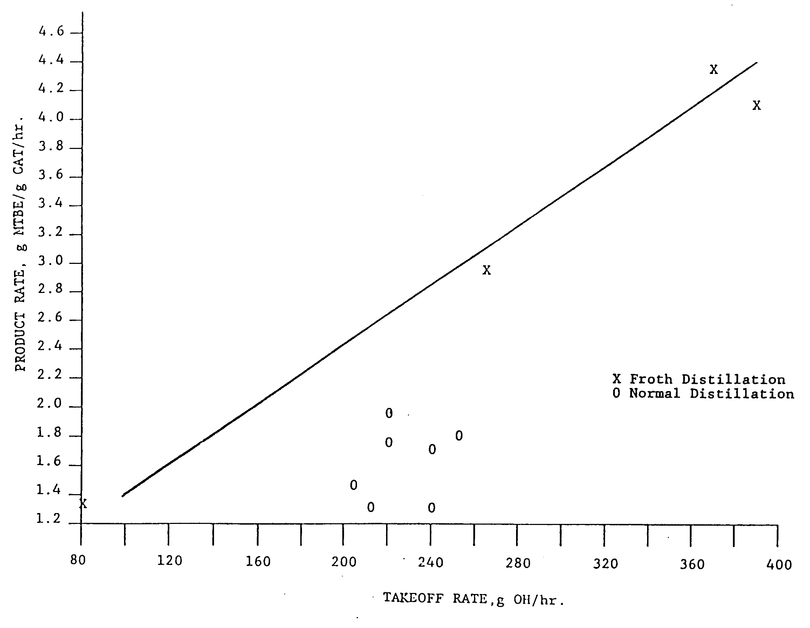

In FIG. 3 a graphical comparison of the two modes of operation is given. As may readily be seen the Froth Mode of operation produced larger amounts of MTBE/g catalyst/hr proportional to the overhead takeoff rates.

TABLE

| RUN NO. | 1 | 2 | 3 | 4 | 5 | 6 |
|---|---|---|---|---|---|---|
| Feed, g/hr | 681.00 | 454.00 | 454.00 | 726.40 | 227.00 | 408.60 |
| MeOH/TBA, Mole ratio | 2.87 | 2.87 | 2.87 | 2.87 | 1.48 | 1.48 |
| Catalyst Zone Temp.,°C(°F) | 66(151.00) | 67(153.00) | 79(175.00) | 79(175.00) | 81(178.00) | 82(180.00) |
| Tower OH Press.,KPa(psig) | 0(0.00)) | 0(0.00) | 89.63(13.00) | 89.63(13.00) | 96.53(14.00) | 96.53(14.00) |
| TBA Conv., % | 20.30 | 28.11 | 36.11 | 26.08 | 39.83 | 28.79 |
| Prod. Rate, g MTBE/g cat/hr | 1.45 | 1.33 | 1.76 | 1.98 | 1.31 | 1.71 |
| Froth, dP % of liq. filled | about 0 | 0 | 0 | 0 | 0 | 0 |
| OH Takeoff Rate,g/hr | 208.00 | 216.00 | 219.00 | 217.00 | 239.00 | 237.00 |

| RUN NO. | 7 | 8 | 9 | 10 | 11 |
|---|---|---|---|---|---|
| Feed, g/hr | 681.00 | 1135.00 | 635.00 | 681.00 | 454.00 |
| MeOH/TBA, Mole ratio | 1.48 | 1.48 | 1.48 | 1.48 | 1.48 |
| Catalyst Zone Temp.,°C(°F) | 81(178.00) | 78(173.00) | 80(177.00) | 77.7(172.00) | 76(169.00) |
| Tower OH Press., psig | 89.63(13.00)) | 96.53(14.00) | 96.53(14.00) | 96.53(14.00) | 96.53(14.00) |
| TBA Conv., % | 18.19 | 26.60 | 45.10 | 29.90 | 20.36 |
| Prod. Rate, g MTBE/g cat/hr | 1.80 | 4.38 | 4.16 | 2.96 | 1.34 |
| Froth, dP % of liq. filled | 0 | 71.00 | 73.00 | 79.00 | 53.00 |
| OH Takeoff Rate,g/hr | 255.00 | 370.00 | 393.00 | 264.00 | 80.00 |

EP 0 485 078 B1

**Claims**

1.  A process for the production of methyl tertiary butyl ether from the reaction of methanol and tertiary butyl alcohol comprising the steps of:

    (a) feeding a feed mixture containing methanol and tertiary butyl alcohol into a feed zone of a distillation column reactor;
    (b) concurrently in said distillation column reactor

        (i) contacting said feed mixture with an acid ion exchange resin catalyst as a distillation structure component in a distillation reaction zone, thereby reacting at least a portion of said methanol and said tertiary butyl alcohol to form a reaction mixture containing methyl tertiary butyl ether, water, unreacted methanol and unreacted tertiary butyl alcohol, and
        (ii) separating said methyl tertiary butyl ether and said unreacted methanol from said unreacted tertiary butyl alcohol and said water by fractional distillation;

    (c) withdrawing said methyl tertiary butyl ether and said unreacted methanol from said distillation column reactor as overheads; and
    (d) withdrawing said unreacted tertiary butyl alcohol and said water from said distillation column reactor as bottoms
    and characterized in that a continuous liquid level is maintained in said distillation reaction zone.

2.  The process according to claim 1 wherein the overhead pressure is between 25.33 and 2533 kPa (0.25 and 25 atmospheres).

3.  The process according to claim 1 or claim 2 wherein the temperature in said distillation reaction zone is between 4.4 and 204.4 °C (40 and 400 °F).

4.  The process according to any preceding claim wherein said feed zone is below said distillation reaction zone.

5.  The process according to any preceding claim wherein said liquid level covers the catalyst bed.

6.  The process according to any preceding claim wherein the overhead pressure is between 0 and 137.8 kPa (0 and 20 psig).

7.  The process according to any preceding claim wherein the temperature in said distillation reaction zone is between 65.6 and 82.2°C (150 and 180°F).

8.  The process according to any preceding claim wherein the molar ratio of methanol to tertiary butyl alcohol in said feed mixture is between 1:1 and 3:1.

**Patentansprüche**

1.  Verfahren zur Herstellung von Methyl-tert.-Butylether aus der Reaktion von Methanol und tert.-Butylalkohol, welches die Schritte umfaßt:

    (a) ein Beschickungsgemisch von Methanol und tert.-Butylalkohol in eine Beschickungszone eines Destillationskolonnenreaktors einzubringen,
    (b) gleichzeitig in dem Destillationskolonnenreaktor

        (i) das Beschickungsgemisch mit einem sauren Ionenaustauscherharzkatalysator als eine Destillationsstrukturkomponente in einer Destillationsreaktionszone in Kontakt zu bringen, wobei mindestens ein Teil des Methanols und des tert.-Butylalkohols unter Bildung eines Reaktionsgemisches, das Methyl-tert.-Butylether, Wasser, nicht umgesetztes Methanol und nicht umgesetzten tert.-Butylalkohol enthält, umgesetzt wird,
        (ii) den Methyl-tert.-Butylether und das nicht umgesetzte Methanol von dem nicht umgesetzten tert.-Butylalkohol und dem Wasser durch fraktionierte Destillation zu trennen,

(c) den Methyl-tert.-Butylether und das nicht umgesetzte Methanol aus dem Destillationskolonnenreaktor als Kopfprodukte zu entfernen, und

(d) den nicht umgesetzten tert.-Butylalkohol und das Wasser aus dem Destillationskolonnenreaktor als Bodenprodukte zu entfernen, und dadurch gekennzeichnet, daß in der Destillationsreaktionszone eine gleichbleibende Flüssigkeitsmenge aufrechterhalten wird.

2. Verfahren nach Anspruch 1, wobei der Druck im Vorlauf zwischen 25,33 und 2533 kPa (0,25 und 25 Atm) beträgt.

3. Verfahren nach Anspruch 1 oder Anspruch 2, wobei die Temperatur in der Destillationsreaktionszone zwischen 4,4 und 204,4°C (40° und 400°F) liegt.

4. Verfahren nach einem der vorhergehenden Ansprüche, wobei der Zuführbereich unterhalb der Destillationsreaktionszone liegt.

5. Verfahren nach einem der vorhergehenden Ansprüche, wobei die Flüssigkeitsmenge das Katalysatorbett bedeckt.

6. Verfahren nach einem der vorhergehenden Ansprüche, wobei der Vorlaufdruck zwischen 0 und 137,8 kPa (0 und 20 Psig) liegt.

7. Verfahren nach einem der vorhergehenden Ansprüche, wobei die Temperatur in der Destillationsreaktionszone zwischen 65,6° und 82,2°C (150° und 180°F) liegt.

8. Verfahren nach einem der vorhergehenden Ansprüche, wobei das Molverhältnis von Methanol zu tert.-Butylalkohol in dem Beschickungsgemisch zwischen 1:1 und 3:1 liegt.

**Revendications**

1. Procédé de production de méthyl-ter-butyl éther à partir de la réaction du méthanol et de l'alcool ter-butylique, comprenant les étapes :

(a) d'introduction d'un mélange d'alimentation contenant du méthanol et de l'alcool ter-butylique, dans la zone d'alimentation d'un réacteur-colonne de distillation ;
(b) et concurremment, dans ledit réacteur-colonne de distillation,

- (i) de mise en contact dudit mélange d'alimentation avec un catalyseur résine échangeuse d'ions en tant que composant de la structure de distillation, dans une zone de réaction-distillation, en faisant ainsi réagir au moins une partie dudit méthanol et dudit alcool ter-butylique pour former un mélange réactionnel contenant du méthyl-ter-butyl éther, de l'eau, du méthanol non transformé, et de l'alcool ter-butylique non transformé, et
- (ii) de séparation dudit méthyl-ter-butyl éther et dudit méthanol non transformé, d'une part, dudit alcool ter-butylique et de ladite eau d'autre part, par distillation fractionnée ;

(c) de soutirage dudit méthyl-ter-butyl éther et dudit méthanol non transformé, à partir dudit réacteur-colonne de distillation, sous forme de fractions de tête ; et
(d) de soutirage dudit alcool ter-butylique non transformé et de ladite eau, à partir dudit réacteur-colonne de distillation, sous forme de fractions de queue,
et caractérisé en ce qu'un niveau constant de liquide est maintenu en continu dans ladite zone de réaction-distillation.

2. Procédé selon la revendication 1, dans lequel la pression en tête est comprise entre 25,33 et 2.533 kPa (0,25 et 25 atmosphères).

3. Procédé selon la revendication 1, ou la revendication 2, dans lequel la température dans ladite zone de réaction-distillation est comprise entre 44 et 204,4°C (40 et 400°F).

4. Procédé selon l'une quelconque des revendications précédentes, dans lequel ladite zone d'alimentation est au dessous de ladite zone de réaction-distillation.

5. Procédé selon l'une quelconque des revendications précédentes, dans lequel ledit niveau de liquide recouvre le lit catalytique.

6. Procédé selon l'une quelconque des revendications précédentes, dans lequel la pression en tête est comprise entre 0 et 137,8 kPa (0 et 20 psig).

7. Procédé selon l'une quelconque des revendications précédentes, dans lequel la température dans ladite zone de réaction-distillation est comprise entre 65,6 et 82,2°C (150 et 180°F).

8. Procédé selon l'une quelconque des revendications précédentes, dans lequel le ratio molaire entre le méthanol et l'alcool ter-butylique dans ledit mélange d'alimentation, est compris entre 1/1 et 3/1.

FIG. 1

MTBE
MeOH
iC$_4$=

MeOH
TBA

iC$_4$=

MTBE
MeOH

REFLUX

TBA
H$_2$O

MeOH
TBA

iC$_4$=

MTBE

MeOH
TBA

H$_2$O
MTBE
iC$_4$=

REFLUX

TBA
H$_2$O

Fig. 2

PRODUCT RATE, g MTBE/g CAT/hr.

4.6
4.4
4.2
4.0
3.8
3.6
3.4
3.2
3.0
2.8
2.6
2.4
2.2
2.0
1.8
1.6
1.4
1.2

80    120    160    200    240    280    320    360    400

TAKEOFF RATE, g OH/hr.

X Froth Distillation
0 Normal Distillation

EP 0 485 078 B1